# EUROPÄISCHE PATENTSCHRIFT

(11) **EP 1 382 669 B1**
(45) Veröffentlichungstag und Bekanntmachung des Hinweises auf die Patenterteilung: **31.01.2007**
(21) Anmeldenummer: 03014010.7
(22) Anmeldetag: 21.06.2003
(51) Int. Cl.: C12M 1/22

(54) **Zellkultureinrichtung mit Diamantbeschichtungsaufwachsoberfläche**
Cell culture device with a surface coated with polycrystalline diamond
Dispositif pour la culture de cellules comprenant un fond tapissé de diamant polycristallin

(30) Priorität: 19.07.2002 AT 10972002
(43) Veröffentlichungstag der Anmeldung: 21.01.2004
(73) Patentinhaber: Physikalisches Büro Steinmüller GmbH, 6074 Rinn/Aldrans (AT)
(72) Erfinder: Steinmüller, Doris, Dr., 6074 Rinn/Aldrans (AT); Steinmüller, Detlef, Dr., 6074 Rinn/Aldrans (AT)
(74) Vertreter: Hefel, Herbert

(56) Entgegenhaltungen:
- FR-A- 1 255 356
- GB-A- 2 221 920
- US-B1- 6 398 815
- SCHROEDER A ET AL: "Titanium containing amorphous hydrogenated carbon films (a-C: H/Ti): surface analysis and evaluation of cellular reactions using bone marrow cell cultures in vitro." BIOMATERIALS, (2000 MAR) 21 (5) 449-56. , XP004185541
- LU L.; JONES M.W.; WU R.L.C.: 'DIAMOND-LIKE CARBON AS BIOLOGICAL COMPATIBLE MATERIAL FOR CELL CULTURE AND MEDICAL APPLICATION' BIO-MEDICAL MATERIALS AND ENGINEERING Bd. 3, Nr. 4, 1993, IOS PRESS, AMSTERDAM, NL, Seiten 223 - 228, XP008048975

## Beschreibung

Die Erfindung betrifft eine Zellkultureinrichtung zur Vermehrung von Zellen mit einer an einem Aufwachssubstrat vorgesehenen Aufwachsoberfläche für die zu vermehrenden Zellen, wobei die Aufwachsoberfläche von der Oberfläche einer kohlenstoffhaltigen Beschichtung gebildet wird.

Die Erfolge der medizinischen Forschung in den letzten Jahrzehnten sind enorm. Heute stehen wir an einem Punkt in der Geschichte der Entwicklung, an dem der Ersatz von geschädigten Körperteilen oder die Wiederherstellung gestörter Körperfunktionen ohne die ethischen Vorbehalte und Bereitstellungsprobleme traditioneller Transplantationsmethoden in greifbare Nähe rücken.

In der Gewebetechnologie fließen biotechnologisches Wissen und klinische Medizin zusammen. Die Gewebetechnologie eröffnet die Chance, Millionen von Patienten weltweit eine dringend benötigte Therapie zu ermöglichen.

Am Beispiel der autologen Periodontalzellimplantation stellt sich die Methode der Gewebetechnologie folgendermaßen dar:

Im Rahmen einer enoralen Operation (Zahnwurzelcurretage) wird eine kleine Gewebeprobe (Biopsie) des Zahnhalteapparates (Periodont) entnommen.

Das bei der Biopsie entnommene Material wird an das gewebetechnologische Labor gesandt. Unter streng aspetischen (absolut keimfreien) Bedingungen werden die in der Gewebeprobe enthaltenen Zellen isoliert, mit Nährstoffen versetzt und in der Zellkultur vermehrt. Die Ansiedlung der Zellkultur dauert 1 bis 6 Wochen, während derer sich die Zahl der entnommenen Zellen um ein Vielfaches vermehrt. Wenn die Zellkultur abgeschlossen ist und sich eine ausreichende Zahl neuer Zellen gebildet hat, werden diese an die Klinik zur Einbringung in den Körper, beispielsweise durch Implantation zurückgeschickt.

Die gewebetechnologisch hergestellten Zellen können beispielsweise im Rahmen eines zweiten chirurgischen Eingriffes in das Zielgebiet eingebracht werden und bilden neues Gewebe.

Typische weitere Anwendungsbereiche der Gewebetechnik sind unter anderem Erkrankungen der Haut (Verbrennungen, Druckgeschwüre, Ulcus usw.), der Knochen, Knorpel, Gelenke, Blutgefäße, Leber usw..

Um die gewünschten Zellen, unter anderem auch Stammzellen, zu vermehren, werden unterschiedliche Aufwachsoberflächen in unterschiedlichen Geometrien, insbesondere zur Maximierung ihrer Gesamtfläche, eingesetzt, auf welchen diese Zellen aufwachsen können. Unter anderem werden verwendet Titan, Titanlegierungen, Keramiken, Saphir, Glas, Kunststoffe und Polymere. Es ist bereits bekannt, die Oberflächen dieser Materialien mit verschiedenen Methoden aufzurauen, um das Anhaften und das Wachstum der Zellen zu verbessern oder überhaupt erst zu ermöglichen. Weiters wurden zu diesem Zweck bereits unterschiedliche Beschichtungen der Oberflächen eingesetzt, beispielsweise Beschichtungen mit organischen Materialien, Kohlefaserkomponenten, diamantartigem Kohlenstoff oder sogenannten a-C:H-Schichten.

Eine Zellkultureinrichtung der eingangs genannten Art ist insbesondere aus der GB 2 221 920 A bekannt. Die Aufwachsoberfläche des Zellkulturapparats ist hierbei mit einer kohlenstoffhaltigen Beschichtung versehen, welche von diamantartigem Kohlenstoff, pyrolytischem Kohlenstoff, glasartigem Kohlenstoff und/oder turbostratischem (turbostratic) Kohlenstoff gebildet wird.

Aufgabe der Erfindung ist, eine verbesserte Zellkultureinrichtung der eingangs genannten Art bereitzustellen und erfindungsgemäß gelingt dies durch eine Zellkultureinrichtung mit den Merkmalen des Patentanspruchs 1.

Überraschenderweise hat es sich gezeigt, daß bei einer Beschichtung des Zellwachstumssubstrats mit einer polykristallinen Diamantschicht eine Aufwachsoberfläche erhalten wird, welche für eine Vielzahl von unterschiedlichen Zelltypen zu einem verbesserten Zellwachstum führt. Insbesondere wurde ein schnelleres Aufwachsen einer Zellschicht auf der Aufwachsoberfläche beobachtet. Auch wurde die Ausbildung von Zellschichten mit verbesserten Eigenschaften beobachtet.

Diamantbeschichtungen werden üblicherweise vor allem für hochstrapazierte Oberflächen eingesetzt, um deren Haltbarkeit zu verbessern. Aufgrund der ansprechenden Oberfläche von diamantbeschichteten Materialien wurden Diamantbeschichtungen auch bereits für dekorative Artikel eingesetzt. Im Hinblick auf deren Haltbarkeit wurden Diamantschichten auch bereits bei hochstrapazierten Gelenkflächen von Implantaten eingesetzt, wie dies beispielsweise in der US 6,398,815 B1 beschrieben ist. Für die zur Verankerung im Knochen dienenden Oberflächen sind in dieser Schrift unterschiedliche Materialien vorgeschlagen, neben Metallgittern, porösem Metall, plasmabeschichtetem Metall usw. auch poröser Diamant, wobei diese Materialien ein Einwachsen von Knochen ermöglichen.

Zwischen polykristallinen Diamantschichten und anderen kohlenstoffhaltigen Schichten bestehen wesentliche Unterschiede. Herstellung und Eigenschaften von diamantartigen Kohlenstoffschichten, auch im Vergleich zu Diamantschichten, sind beispielsweise behandelt in: "Development and Status of Diamondlike Carbon", Alfred Grill und Bernard S. Meyerson in "Synthetic Diamond: Emerging CVD Science and Technology", Karl E. Spear and John P. Dismukes, John Wiley & Sons, Inc. 1994. Diamantartige Kohlenstoffschichten bestehen im Gegensatz zu polykristallinen Diamantschichten aus im wesentlichen amorphem Kohlenstoff, wobei diese Schichten eine relativ große Härte aufweisen (daher auch die Bezeichnung "diamantartig"). Die Herstellung von gleichförmigen Schichten über einen relativ großen Bereich eines Substrats ist hierbei verhältnismäßig leicht möglich.

Die Herstellung von polykristallinen Diamantschichten ist demgegenüber mit einem vergleichsweise höheren technischen Aufwand verbunden. Beispielsweise geht die Herstellung von solchen polykristallinen Diamantschichten aus folgenden Veröffentlichungen hervor: EP 0 297 845 und US 5,378,285 (Plasmaverfahren), US 6,383,288 und **JP 2 092 895** (hot filament Verfahren), EP 0 297 845 A1 und US 6,200,652 (Hybrid-Verfahren).

Weitere Vorteile und Einzelheiten der Erfindung werden im folgenden anhand der beiliegenden Zeichnung erläutert. In dieser zeigt:

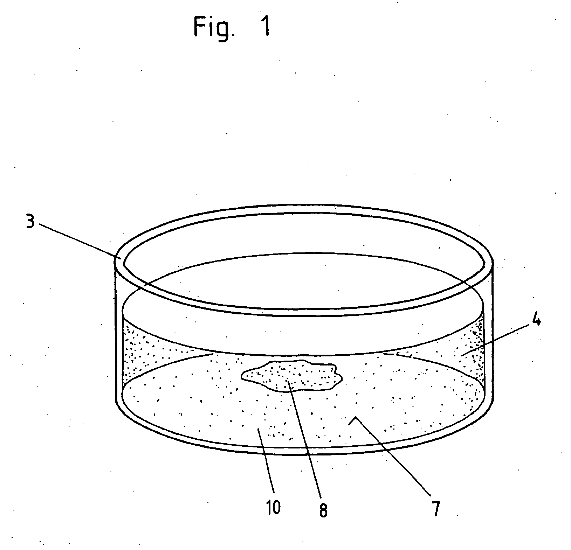

- Fig. 1: eine schematische Darstellung einer erfindungsgemäßen Zellkultureinrichtung.

Die in Fig. 1 gezeigte Zellkultureinrichtung zur Vermehrung von Zellen ist als Schale 3 ausgebildet, die mit einer Nährflüssigkeit 4 teilweise gefüllt ist, in welche zu vermehrende Zellen eingebracht sind.

In einer Ausführungsform der Erfindung kann das Material der Schale 3, beispielsweise im Bereich ihres Bodens 10 an der mit der Nährflüssigkeit 4 in Kontakt stehenden Oberfläche mit einer polykristallinen Diamantbeschichtung versehen sein. Zellen 8 können somit direkt auf dieser eine Aufwachsoberfläche 7 bildenden Oberfläche des Bodens 10 aufgewachsen werden, beispielsweise um nach Ausbildung einer ausreichenden Zahl von solchen Zellen bei einer Operation zum Einsatz zu kommen, bei welcher sie in den menschlichen oder tierischen Körper in geeigneter Form eingebracht werden.

Als Beispiel für eine Operation, im Zusammenhang mit welcher die Erfindung zum Einsatz kommen kann, wird die bereits beschriebene autologe Periodontalzellimplantation genannt: Im Rahmen einer enoralen Operation (Zahnwurzelcurretage) wird zunächst eine kleine Gewebeprobe (Biopsie) des Zahnhalteapparates (Periodont) entnommen. Das bei der Biopsie entnommene Material wird an das gewebetechnologische Labor gesandt. Unter streng aseptischen (absolut keimfreien) Bedingungen werden die in der Gewebeprobe enthaltenen Zellen isoliert, mit Nährstoffen versetzt und in der Zellkultur vermehrt. Die Aufwachsoberfläche der Wachstumsschale zum Aufwachsen der Zellen kann hierbei erfindungsgemäß als Oberfläche einer polykristallinen Diamantbeschichtung ausgebildet sein. Die Ansiedlung der Zellkultur dauert 2 bis 6 Wochen, während derer sich die Zahl der entnommenen Zellen um ein Vielfaches vermehrt. Wenn die Zellkultur abgeschlossen ist und sich eine ausreichende Zahl neuer Zellen gebildet hat, werden diese an die Klinik zur Einbringung in den Körper, beispielsweise durch Implantation zurückgeschickt.

Die gewebetechnologisch hergestellten Zellen können im Rahmen eines zweiten chirurgischen Eingriffes in das Zielgebiet in den Körper eingebracht werden und bilden neues Gewebe.

Durch die erfindungsgemäße Ausbildung der Aufwachsoberfläche der Wachstumsschale oder eines in die Wachstumsschale eingebrachten, beispielsweise plattenförmigen Kulturkörpers wird das Zellwachstum wesentlich verbessert, insbesondere stark beschleunigt.

Das Grundmaterial des Zellaufwachssubstrates kann beispielsweise von einem Metall wie Titan, einer Titanlegierung oder einem Hartmetall, z. B. Wolfram-, Titan,- oder Tantalcarbid gebildet werden. Weiters kann dieses Grundmaterial u. a. von einem Kunststoff, beispielsweise in Form eines Polymers, von Saphir, Keramiken, Glas oder Hartglas gebildet werden. Grundsätzlich ist es auch denkbar und möglich, zwischen dem Grundmaterial und der Diamantbeschichtung eine oder mehrere Zwischenschichten einzubringen, beispielsweise aus einem organischen Material oder in Form einer herkömmlichen CVD- oder PVD-Beschichtung, z. B. Titancarbid.

Das Zellaufwachssubstrat wird von einem Teil einer Zellkultureinrichtung zur Vermehrung von Zellen gebildet, beispielsweise dem Boden einer Zellwachstumsschale oder einem in die Zellwachstumsschale eingebrachten, insbesondere plattenförmigen Kulturkörper. Das auf dieses Zellaufwachssubstrat aufgewachsene Gewebe wird von der Aufwachsoberfläche wieder abgelöst und anschließend im lebenden Körper eingesetzt, beispielsweise im Zusammenhang mit Haut-, Knorpel- oder Knochengewebe.

Unter anderem können auf ein erfindungsgemäßes Zellwachstumssubstrat humane und nicht humane Zellen, Tumorzellen, Epitelzellen, mesenchymale Zellen oder pränatale, postnatale oder adulte Stammzellen aufgewachsen werden.

Die Dicke der Diamantbeschichtung kann beispielsweise im Bereich zwischen 5 nm und 50 um liegen. Bevorzugt sind polykristalline Diamantschichten, deren Korngrößen im Nanometerbereich liegen, insbesondere im Bereich von weniger als 100 nm. Bevorzugt ist ein Wert der durchschnittlichen Korngröße von weniger als 50 nm. Auch ein Wert der durchschnittlichen Korngröße von weniger als 25 nm ist denkbar und möglich.

Besonders gut geeignet sind sehr reine polykristalline Diamantschichten, deren sp³-Hybridisierung mehr als 80%, vorzugsweise mehr als 90% beträgt.

## Patentansprüche

1. Zellkultureinrichtung zur Vermehrung von Zellen (8) mit einer an einem Zellaufwachssubstrat vorgesehenen Aufwachsoberfläche (7) für die zu vermehrenden Zellen (8), wobei die Aufwachsoberfläche (7) von der Oberfläche einer kohlenstoffhaltigen Beschichtung gebildet wird, **dadurch gekennzeichnet, daß** die Aufwachsoberfläche (7) von der Oberfläche einer polykristallinen Diamantbeschichtung gebildet wird.

2. Zellkultureinrichtung nach Anspruch 1, **dadurch gekennzeichnet, daß** zwischen dem die Diamantbeschichtung tragenden Grundmaterial des Zellaufwachssubstrats und der Diamantbeschichtung eine Zwischenschicht aus anorganischem oder organischem Material vorgesehen ist.

3. Zellkultureinrichtung nach Anspruch 1 oder Anspruch 2, **dadurch gekennzeichnet, daß** die durchschnittliche Korngröße der polykristallinen Diamantbeschichtung weniger als 100 nm, vorzugsweise weniger als 50 nm beträgt.

4. Zellkultureinrichtung nach einem der Ansprüche 1 bis 3, **dadurch gekennzeichnet, dass** die Zellkultureinrichtung eine Nährflüssigkeit enthaltende Zellwachstumsschale (3) aufweist.

5. Zellkultureinrichtung nach Anspruch 4, **dadurch gekennzeichnet, dass** die Zellwachstumsschale (3) selbst das Zellaufwachssubstrat darstellt.

6. Zellkultureinrichtung nach Anspruch 4 oder Anspruch 5, **dadurch gekennzeichnet, dass** das Zellaufwachssubstrat ein in die Zeilwachstumsschale (3) eingebrachter Kulturkörper ist, der vorzugsweise plattenförmig ausgebildet ist.

7. Zellkultureinrichtung nach einem der Ansprüche 1 bis 6, **dadurch gekennzeichnet, daß** die zu vermehrenden, aufgewachsenen Zellen Tumorzellen sind.

8. Zellkultureinrichtung nach einem der Ansprüche 1 bis 6, **dadurch gekennzeichnet, daß** die zu vermehrenden aufgewachsenen Zellen Epitelzellen sind.

9. Zellkultureinrichtung nach einem der Ansprüche 1 bis 6, **dadurch gekennzeichnet, daß** die zu vermehrenden aufgewachsenen Zellen mesenchymale Zellen sind.

10. Zellkultureinrichtung nach einem der Ansprüche 1 bis 5, **dadurch gekennzeichnet, daß** die zu vermehrenden aufgewachsenen Zellen prenatale, postnatale oder adulte Stammzellen sind.

11. Zellkultureinrichtung nach einem der Ansprüche 1 bis 10, **dadurch gekennzeichnet, dass** die polykristalline Diamantschicht eine sp³-Hybridisierung von mehr als 80%, vorzugsweise mehr als 90% aufweist.

## Claims

1. Cell culture device for the proliferation of cells (8), having a growth surface (7), provided on a cell growth substrate, for the cells (8) to be proliferated, the growth surface (7) being formed by the surface of a carbon-containing coating, **characterised in that** the growth surface (7) is formed by the surface of a polycrystalline diamond coating.

2. Cell culture device according to Claim 1, **characterised in that** an intermediate layer made of inorganic or organic material is provided between the diamond-coating-bearing base material of the cell growth substrate and the diamond coating.

3. Cell culture device according to Claim 1 or Claim 2, **characterised in that** the average grain size of the polycrystalline diamond coating is less than 100 nm, preferably less than 50 nm.

4. Cell culture device according to one of Claims 1 to 3, **characterised in that** the cell culture device has a nutrient-liquid-containing cell growth dish (3).

5. Cell culture device according to Claim 4, **characterised in that** the cell growth dish (3) itself constitutes the cell growth substrate.

6. Cell culture device according to Claim 4 or Claim 5, **characterised in that** the cell growth substrate is a preferably plate-shaped culture body which is introduced into the cell growth dish (3).

7. Cell culture device according to one of Claims 1 to 6, **characterised in that** the grown cells to be proliferated are tumour cells.

8. Cell culture device according to one of Claims 1 to 6, **characterised in that** the grown cells to be proliferated are epithelial cells.

9. Cell culture device according to one of Claims 1 to 6, **characterised in that** the grown cells to be proliferated are mesenchymal cells.

10. Cell culture device according to one of Claims 1 to 5, **characterised in that** the grown cells to be proliferated are prenatal, postnatal or adult stem cells.

11. Cell culture device according to one of Claims 1 to 10, **characterised in that** the polycrystalline diamond layer has an sp³ hybridisation of more than 80%, preferably more than 90%.

## Revendications

1. Dispositif de culture de cellules pour la multiplication de cellules (8) comprenant une surface de croissance (7) prévue sur un substrat de croissance de cellules pour les cellules (8) à multiplier, la surface de croissance (7) étant formée par la surface d'un revêtement contenant du carbone,
**caractérisé en ce que**
la surface de croissance (7) est formée par la surface d'un revêtement de diamant polycristallin.

2. Dispositif de culture de cellules selon la revendication 1,
**caractérisé en ce qu'**
une couche intermédiaire en matériau anorganique ou organique est prévue entre le matériau de base du substrat de croissance de cellules portant le revêtement de diamant, et le revêtement de diamant.

3. Dispositif de culture de cellules selon la revendication 1 ou 2,
**caractérisé en ce que**
la granulométrie moyenne du revêtement de diamant polycristallin est inférieure à 100 nm, de préférence inférieure à 50 nm.

4. Dispositif de culture de cellules selon l'une des revendications 1 à 3,
**caractérisé en ce que**
le dispositif de culture de cellules présente une cuvette de croissance de cellules (3) contenant un liquide nutritif.

5. Dispositif de culture de cellules selon la revendication 4,
**caractérisé en ce que**
la cuvette de croissance de cellules (3) représente elle-même le substrat de croissance de cellules.

6. Dispositif de culture de cellules selon la revendication 4 ou 5,
**caractérisé en ce que**
le substrat de croissance de cellules est un corps de culture, de préférence en forme de plaque, inséré dans la cuvette de croissance de cellules (3).

7. Dispositif de culture de cellules selon l'une des revendications 1 à 6,
**caractérisé en ce que**
les cellules à multiplier qui se sont développées sont des cellules tumorales.

8. Dispositif de culture de cellules selon l'une des revendications 1 à 6,
**caractérisé en ce que**
les cellules à multiplier qui se sont développées sont des cellules épithéliales.

9. Dispositif de culture de cellules selon l'une des revendications 1 à 6,
**caractérisé en ce que**
les cellules à multiplier qui se sont développées sont des cellules mésenchymateuses.

10. Dispositif de culture de cellules selon l'une des revendications 1 à 5,
**caractérisé en ce que**
les cellules à multiplier qui se sont développées sont des cellules mères prénatales, postnatales ou adultes.

11. Dispositif de culture de cellules selon l'une des revendications 1 à 10,
**caractérisé en ce que**
la couche de diamant polycristallin présente une hybridation sp³ supérieure à 80 %, de préférence supérieure à 90 %.
